# EUROPEAN PATENT APPLICATION

(11) **EP 4 699 571 A1**
(43) Date of publication of application: **25.02.2026**
(21) Application number: 23938075.1
(22) Date of filing: 20.06.2023
(51) Int. Cl.: A61F 2/24

(54) **VALVE DELIVERY DEVICE**

(30) Priority: 25.05.2023 CN 202310598317
(71) Applicant: Kingstronbio (Changshu) Co., Ltd., Changshu Jiangsu 215500 (CN)
(72) Inventor: ZHANG, Bo, Suzhou, Jiangsu 215500 (CN)
(74) Representative: Taor, Simon Edward William
(86) International application number: PCT/CN2023/101316
(87) International publication number: WO 2024/239406

(57) **Abstract**

The present disclosure relates to a valve delivery device. The valve delivery device includes a valve provided with a fixing part and a valve delivery system. The valve delivery system includes a retraction assembly and a wire-locking claw assembly. The retraction assembly includes a fixing claw. One end of the fixing claw is provided with a first clamping part and a second clamping part. A mounting groove is formed between the first clamping part and the second clamping part. The fixing part is located in the mounting groove. The first clamping part and the second clamping part are configured to clamp the fixing part. The wire-locking claw assembly includes a wire-locking claw and a sleeving part, and is sleeved on the fixing claw, thereby realizing control over the valve release and improving the stability of the valve during release.

## Description

### TECHNICAL FIELD

The present disclosure relates to the technical field of medical devices, and in particular relates to a valve delivery device.

### BACKGROUND

With the development of medical technologies, heart valves can be implanted into a human body through surgical procedures to treat heart diseases. A method is to implant a valve into a preset position in the human body through an implantation device and release it. Under an action of its own elasticity, the valve can automatically expand and deploy to achieve functions of support and expansion. However, when the valve detaches from the implantation device and opens up after being released, it is prone to bouncing, leading to the displacement of the valve, affecting the treatment effect, and even causing damage to the human body.

### SUMMARY

The present disclosure provides a valve delivery device for improving the stability of a valve during release.

The present disclosure provides a valve delivery device. The valve delivery device includes a valve provided with a fixing part and a valve delivery system. The valve delivery system includes a retraction assembly and a wire-locking claw assembly. The retraction assembly includes a fixing claw. One end of the fixing claw is provided with a first clamping part and a second clamping part. A mounting groove is formed between the first clamping part and the second clamping part. The fixing part is located in the mounting groove. The first clamping part and the second clamping part are configured to clamp the fixing part. The wire-locking claw assembly includes a wire-locking claw and a sleeving part. The sleeving part is fixed to one end of the wire-locking claw and sleeved on the fixing claw. When the sleeving part is sleeved on the first clamping part and the second clamping part, the valve is in a locked state. When the sleeving part disengages from the first clamping part and the second clamping part, the valve is in a released state.

In a feasible embodiment, along an axial direction of the valve delivery system, a cross-sectional area of at least part of the first clamping part and the second clamping part gradually increases.

In a feasible embodiment, the fixing part includes a connecting part and a snap-fit part. The snap-fit part is located at one end of the connecting part adjacent to the fixing claw. A cross-sectional area of the snap-fit part perpendicular to an axial direction of the valve delivery system is larger than a cross-sectional area of the connecting part.

In a feasible embodiment, the wire-locking claw assembly includes a plurality of wire-locking claws arranged at intervals along a circumferential direction of the valve delivery system. The plurality of wire-locking claws is respectively provided with the sleeving part. The retraction assembly includes a plurality of fixing claws. The valve is provided with a plurality of fixing parts. The fixing parts, the fixing claws and the sleeving parts are arranged in correspondence.

In a feasible embodiment, a size of at least one of the plurality of fixing claws and wire-locking claws in an axial direction of the valve delivery system is different from a size of the other fixing claws and wire-locking claws.

In a feasible embodiment, the valve delivery system includes a claw releasing assembly. The claw releasing assembly includes a claw releasing connecting pipe and a claw releasing handle. The claw releasing connecting pipe has one end connected to the wire-locking claw assembly, and the other end connected to the claw releasing handle.

In a feasible embodiment, the retraction assembly further includes a fixing base and a retraction connecting pipe. An end of the fixing claw not provided with the mounting groove is connected to the fixing base. The claw releasing assembly is sleeved on the retraction connecting pipe, and an end of the retraction connecting pipe is connected to the fixing base.

In a feasible embodiment, the retraction assembly further includes a mounting part. The mounting part is located on a side of the fixing claw not provided with the mounting groove. The mounting part is sleeved on the fixing base and connected to the fixing claw.

In a feasible embodiment, the valve delivery system includes a receiving pipe and a rotating wheel assembly. The receiving pipe is sleeved on the retraction assembly and configured to accommodate the valve. The rotating wheel assembly is configured to control movement of the receiving pipe along an axial direction of the valve delivery system.

In a feasible embodiment, the valve delivery system includes a limit pin. The limit pin is located at an end of the claw releasing connecting pipe away from the wire-locking claw assembly, and is configured to limit the movement of the claw releasing connecting pipe along an axial direction of the valve delivery system.

The present disclosure provides the valve delivery device. The valve delivery device includes the valve provided with the fixing part and the valve delivery system. The valve delivery system includes the retraction assembly and the wire-locking claw assembly. The retraction assembly includes the fixing claw. One end of the fixing claw is provided with the first clamping part and the second clamping part. The mounting groove is formed between the first clamping part and the second clamping part. The fixing part is located in the mounting groove. The first clamping part and the second clamping part are configured to clamp the fixing part. The wire-locking claw assembly includes the wire-locking claw and the sleeving part, and is sleeved on the fixing claw, thereby achieving control the valve release and improving the stability of the valve during release.

It should be understood that the above general description and the following detailed description are merely exemplary and shall not limit the present disclosure.

### BRIEF DESCRIPTION OF DRAWINGS

FIG. 1 is a schematic structural diagram of a valve delivery system according to an embodiment of the present disclosure;
FIG. 2 is a schematic structural diagram of a fixing part according to an embodiment of the present disclosure;
FIG. 3 is a schematic structural diagram of a snap-fit part according to an embodiment of the present disclosure;
FIG. 4 is a schematic structural diagram of a retraction assembly according to an embodiment of the present disclosure;
FIG. 5 is a schematic structural diagram showing cooperation between the fixing part, the fixing claw and the sleeving part provided by the present disclosure; and
FIG. 6 is a schematic diagram of an internal structure of a valve delivery system according to an embodiment of the present disclosure.

### Reference Signs:

1 - Fixing part;
   11 - Connecting part;
   12 - Snap-fit part;
2 - Retraction assembly;
   21 - Fixing claw;
      211 - First clamping part;
      212 - Second clamping part;
      213 - Mounting groove;
   22 - Fixing base;
   23 - Retraction connecting pipe;
   24 - Mounting part;
3 - Wire-locking claw assembly;
   31 - Wire-locking claw;
   32 - Sleeving part;
4 - Claw releasing assembly;
   41 - Claw releasing connecting pipe;
   42 - Claw releasing handle;
5 - Rotating wheel assembly;
6 - Receiving pipe;
7 - Limit pin;
8 - Introducing device.

The accompanying drawings herein are incorporated into and form a part of this specification, illustrate embodiments consistent with the present disclosure, and together with the specification serve to explain the principles of the present disclosure.

### DESCRIPTION OF EMBODIMENTS

In order to better understand technical solutions of the present disclosure, the embodiments of the present disclosure are described in details with reference to the drawings.

It should be clear that the described embodiments are merely part of the embodiments of the present disclosure rather than all of the embodiments. All other embodiments obtained by those skilled in the art without paying creative labor shall fall into the protection scope of the present disclosure.

The terms used in the embodiments of the present disclosure are merely for the purpose of describing specific embodiment, rather than limiting the present disclosure. The terms "a", "an", "the" and "said" in a singular form in the embodiment of the present disclosure and the attached claims are also intended to include plural forms thereof, unless noted otherwise.

It should be understood that the term "and/or" used in the context of the present disclosure is to describe a correlation relation of related objects, indicating that there may be three relations, e.g., A and/or B may indicate only A, both A and B, and only B. In addition, the symbol "/" in the context generally indicates that the relation between the objects in front and at the back of "/" is an "or" relationship.

It should be noted that the positional terms such as "upper", "lower", "left" and "right" described in the embodiments of the present disclosure are described from the perspective shown in the accompanying drawings, and shall not be construed as limiting the embodiments of the present disclosure. In addition, in the context, it should also be understood that when it is mentioned that one element is connected "on" or "under" another element, it can not only be directly connected "on" or "under" another element, but also indirectly connected "on" or "under" another element through an intermediate element.

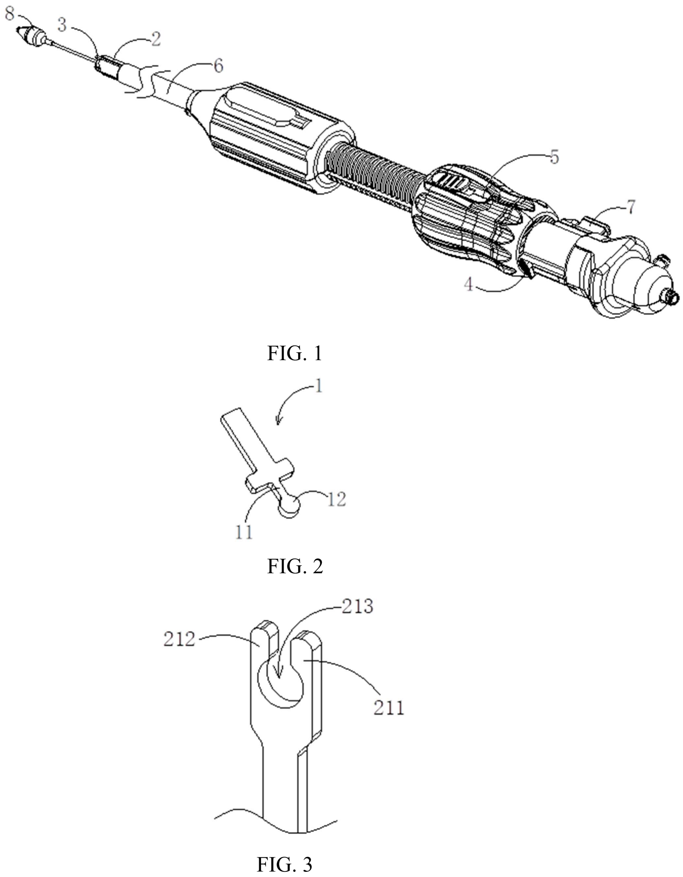

As shown in FIG. 1 to FIG. 3, the present disclosure provides a valve delivery device. The valve delivery device includes a valve provided with a fixing part 1 and a valve delivery system. The valve delivery system includes a retraction assembly 2 and a wire-locking claw assembly 3. The retraction assembly 2 includes a fixing claw 21. One end of the fixing claw 21 is provided with a first clamping part 211 and a second clamping part 212. A mounting groove 213 is formed between the first clamping part 211 and the second clamping part 212. The fixing part 1 is located in the mounting groove 213. The first clamping part 211 and the second clamping part 212 are configured to clamp the fixing part 1. The wire-locking claw assembly 3 includes a wire-locking claw 31 and a sleeving part 32. The sleeving part 32 is fixed to one end of the wire-locking claw 31 and sleeved on the fixing claw 21. When the sleeving part 32 is sleeved on the first clamping part 211 and the second clamping part 212, the valve is in a locked state. When the sleeving part 32 disengages from the first clamping part 211 and the second clamping part 212, the valve is in a released state.

The valve delivery device provided in the present disclosure is configured to implant a valve into a human body. The valve has elasticity and can be installed and accommodated in the valve delivery system, so that the valve can be delivered to a preset position in the human body through the valve delivery system. When the valve is delivered to a preset position in the human body by the valve delivery system, the valve delivery system releases the valve, and the valve deploys under an action of its own elasticity to achieve the effects of support and expansion. One end of the fixing claw 21 adjacent to the valve is provided with a first clamping part 211 and a second clamping part 212. A mounting groove 213 is formed between the first clamping part 211 and the second clamping part 212. The fixing part 1 is mounted in the mounting groove 213 and clamped by the first clamping part 211 and the second clamping part 212. The first clamping part 211 and the second clamping part 212 have elasticity, and the valve can disengage from the mounting groove 213 under the action of its own elasticity. When the sleeving part 32 is sleeved on the first clamping part 211 and the second clamping part 212, the sleeving part 32 can restrict the first clamping part 211 and the second clamping part 212 from moving in a direction away from the fixing part 1, thereby achieving a limiting effect on the fixing part 1 and causing the valve in a locked state. The wire-locking claw assembly 3 and the retraction assembly 2 can respectively move along the axial direction of the valve delivery system. When the retraction assembly 2 or the wire-locking claw assembly 3 moves along the axial direction of the valve delivery system, the sleeving part 32 can disengage from the first clamping part 211 and the second clamping part 212, allowing the first clamping part 211 and the second clamping part 212 to move in the direction away from the fixing part 1. The fixing part 1 can then disengage from the mounting groove 213, and the valve is in a released state to achieve the effects of support and expansion. The first clamping part 211 and the second clamping part 212 can exert a cushioning effect on the fixing part 1, improving the stability of the valve during release and reducing the possibility of human body damage and valve offset when the valve is released.

As shown in FIG. 3, in a feasible embodiment, along an axial direction of the valve delivery system, the cross-sectional area of at least part of the first clamping part 211 and the second clamping part 212 gradually increases.

At least part of the first clamping part 211 and the second clamping part 212 respectively protrudes inward into the mounting groove 213. When the fixing part 1 is located in the mounting groove 213, this can increase the frictional force between the first clamping part 211 and the fixing part 1, and between the second clamping part 212 and the fixing part 1, improving the stability of the fixing part 1 when being limited.

As shown in FIG. 2, in a feasible embodiment, the fixing part 1 includes a connecting part 11 and a snap-fit part 12. The snap-fit part 12 is located at one end of the connecting part 11 adjacent to the fixing claw 21, and a cross-sectional area of the snap-fit part 12 perpendicular to an axial direction of the valve delivery system is larger than a cross-sectional area of the connecting part 11.

The snap-fit part 12 is configured to cooperate with the first clamping part 211 and the second clamping part 212. The snap-fit part 12 has a relatively large cross-sectional area, and the shape of the mounting groove 213 can match the shape of the snap-fit part 12. A crosssection of the snap-fit part 12 can be circular, elliptical and the like, making the snap-fit part 12 more stable when disengaging from the fixing claw 21 and improving the stability of the valve during release.

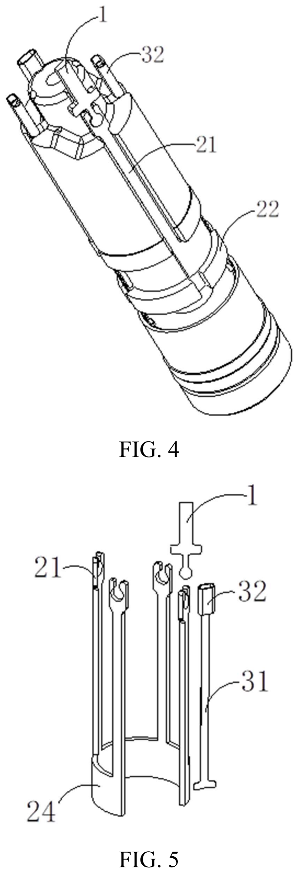

As shown in FIG. 4, in a feasible embodiment, the wire-locking claw assembly 3 includes a plurality of wire-locking claws 31. The plurality of wire-locking claws 31 are arranged at intervals along a circumferential direction of the valve delivery system. The retraction assembly 2 includes a plurality of fixing claws 21. The plurality of wire-locking claws 31 is respectively provided with a sleeving part 32. The valve is provided with a plurality of fixing parts 1. The fixing parts 1 are arranged in correspondence with the sleeving parts 32.

The plurality of fixing claws 21 are arranged along a circumferential direction of the valve delivery system, and their positions correspond to the wire-locking claws 31. The mounting groove 213 of the fixing claw 21 can respectively cooperate with the corresponding sleeving part 32. The plurality of fixing claws 21 can respectively cooperate with the mounting grooves 213 to fix the valve from different directions, improving the stability during fixation. When the valve is released, it can also stably deploy along the circumferential direction of the valve delivery system, further enhancing the stability of the valve during release.

As shown in FIG. 4 and FIG. 5, in a feasible embodiment, according to the valve shape, a size of at least one of the plurality of wire-locking claws 31 and fixing claws 21 in the axial direction of the valve delivery system is different from a size of the other wire-locking claws 31 and fixing claws 21. The length of the fixing claws 21 is configured corresponding to the length of the wire-locking claws 31.

The valve delivery system may be provided with four wire-locking claws 31 and four fixing claws 21. Two adjacent wire-locking claws 31 have a same length, and the corresponding fixing claws 21 also have a same length. When the retraction assembly 2 moves relative to the wire-locking claw assembly 3 along an axial direction of the valve delivery system, the fixing claws 21 can disengage from the sleeving parts 32, allowing the fixing parts 1 to disengage from the mounting grooves 213. Due to different length of the wire-locking claws 31, the fixing parts 1 are released from different positions along the axial direction of the valve delivery system, which reduces the amplitude of deformation of the valve during release, makes the valve release more stable, and lowers the possibility of the valve bouncing when it is released.

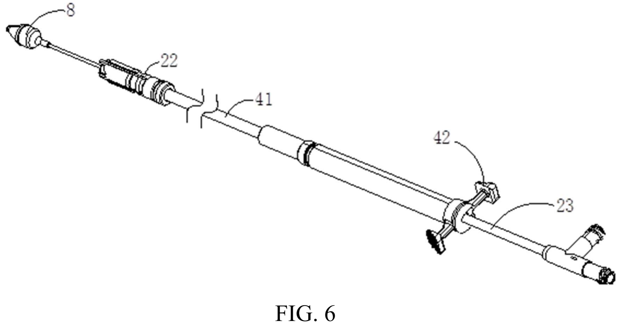

As shown in FIG. 6, in a feasible embodiment, the valve delivery system includes a claw releasing assembly 4. The claw releasing assembly 4 includes a claw releasing connecting pipe 41 and a claw releasing handle 42. One end of the claw releasing connecting pipe 41 is connected to the wire-locking claw assembly 3, and the other end the claw releasing connecting pipe 41 is connected to the claw releasing handle 42. The claw releasing assembly 4 is used for claw releasing to release the valve. The claw releasing connecting pipe can be pulled through the claw releasing handle 42, so that the wire-locking claw assembly 3 is driven to move to achieve claw releasing.

As shown in FIG. 6, in a feasible embodiment, the retraction assembly 2 further includes a fixing base 22 and a retraction connecting pipe 23. An end of the fixing claw 21 not provided with the mounting groove 213 is connected to the fixing base 22. The claw releasing assembly 4 is sleeved on the retraction connecting pipe 23, and one end of the retraction connecting pipe 23 is connected to the fixing base 22 for controlling movement of the retraction assembly 2.

As shown in FIG. 5, in a feasible embodiment, the retraction assembly 2 further includes a mounting part 24. The mounting part 24 is located on a side of the fixing claw 21 not provided with the mounting groove 213, and the mounting part 24 is sleeved on the fixing base 22 and connected to the fixing claw 21. When the retraction assembly 2 includes a plurality of fixing claws 21, the mounting part 24 can drive the plurality of fixing claws 21 to move simultaneously with the movement of the fixing base 22, thereby realizing the release of the plurality of fixing parts 1 and improving the stability of the valve during release.

As shown in FIG. 1, in a feasible embodiment, the valve delivery system includes a receiving pipe 6 and a rotating wheel assembly 5. The receiving pipe 6 is sleeved on the retraction assembly 2 and configured to accommodate the valve, and the rotating wheel assembly 5 is configured to control the movement of the receiving pipe 6 along the axial direction of the valve delivery system.

The receiving pipe 6 is located at an outermost side of the valve delivery system. The rotating wheel assembly 5 is connected by threads and can move along the axial direction of the valve delivery system. An outer pipe can play a role in accommodating and fixing the valve, movement of the outer pipe driven by moving the rotating wheel can release the limit on the valve, the valve can be released. One end of the valve delivery system provided with the valve is further provided with an introducing device 8. The introducing device 8 has a conical tip to facilitate entry into the human body.

As shown in FIG. 1, in a feasible embodiment, the valve delivery system includes a limit pin 7. The limit pin 7 is located at one end of the claw releasing connecting pipe 41 away from the wire-locking claw assembly 3, and is configured to limit the movement of the claw releasing connecting pipe 41 along the axial direction of the valve delivery system. The limit pin 7 abuts against the claw releasing handle 42, thereby restricting the movement of the claw releasing handle 42 along the axial direction of the valve delivery system, achieving a locking effect on the claw releasing assembly and reducing the possibility of faulty operation.

The present disclosure provides a valve delivery device. The valve delivery device includes a valve provided with a fixing part 1 and a valve delivery system. The valve delivery system includes a retraction assembly 2 and a wire-locking claw assembly 3. The retraction assembly 2 includes a fixing claw 21. One end of the fixing claw 21 is provided with a first clamping part 211 and a second clamping part 212. A mounting groove 213 is formed between the first clamping part 211 and the second clamping part 212. The fixing part 1 is located in the mounting groove 213. The first clamping part 211 and the second clamping part 212 are configured to clamp the fixing part 1. The wire-locking claw assembly 3 includes a wire-locking claw 31 and a sleeving part 32. The sleeving part 32 is fixed to one end of the wire-locking claw 31 and sleeved on the fixing claw 21. When the sleeving part 32 is sleeved on the first clamping part 211 and the second clamping part 212, the valve is in a locked state. When the sleeving part 32 disengages from the first clamping part 211 and the second clamping part 212, the valve is in a released state, thereby achieving control the valve release and improving the stability of the valve during release.

The above descriptions are only some embodiments of the present disclosure and are not intended to limit the present disclosure. For those skilled in the art, various modifications and changes may be made to the present disclosure. Any modification, equivalent replacement, improvement and the like, made within the spirit and principles of the present disclosure shall be included in the scope of the present disclosure.

## Claims

1. A valve delivery device, comprising: a valve provided with a fixing part (1), and a valve delivery system,
wherein the valve delivery system comprises:
a retraction assembly (2), wherein the retraction assembly (2) comprises a fixing claw (21), one end of the fixing claw (21) is provided with a first clamping part (211) and a second clamping part (212), a mounting groove (213) is formed between the first clamping part (211) and the second clamping part (212), the fixing part (1) is located in the mounting groove (213), and the first clamping part (211) and the second clamping part (212) are configured to clamp the fixing part (1); and
a wire-locking claw assembly (3), wherein the wire-locking claw assembly (3) comprises a wire-locking claw (31) and a sleeving part (32), the sleeving part (32) is fixed to one end of the wire-locking claw (31) and sleeved on the fixing claw (21);
wherein, when the sleeving part (32) is sleeved on the first clamping part (211) and the second clamping part (212), the valve is in a locked state; and
when the sleeving part (32) disengages from the first clamping part (211) and the second clamping part (212), the valve is in a released state.

2. The valve delivery device according to claim 1, wherein along an axial direction of the valve delivery system, a cross-sectional area of at least part of the first clamping part (211) and the second clamping part (212) gradually increases.

3. The valve delivery device according to claim 1, wherein the fixing part (1) comprises a connecting part (11) and a snap-fit part (12), the snap-fit part (12) is located at one end of the connecting part (11) adjacent to the fixing claw (21), and a cross-sectional area of the snap-fit part (12) perpendicular to an axial direction of the valve delivery system is larger than a cross-sectional area of the connecting part (11).

4. The valve delivery device according to claim 1, wherein the wire-locking claw assembly (3) comprises a plurality of wire-locking claws (31) arranged at intervals along a circumferential direction of the valve delivery system, the plurality of wire-locking claws (31) is respectively provided with a plurality of sleeving parts (32), the retraction assembly (2) comprises a plurality of fixing claws (21), the valve is provided with a plurality of fixing parts (1), and the fixing parts (1), the fixing claws (21) and the sleeving parts (32) are arranged in correspondence.

5. The valve delivery device according to claim 4, wherein a size of at least one of the plurality of fixing claws (21) and wire-locking claws (31) in an axial direction of the valve delivery system is different from a size of the other fixing claws (21) and wire-locking claws (31).

6. The valve delivery device according to any one of claims 1 to 5, wherein the valve delivery system comprises a claw releasing assembly (4), the claw releasing assembly (4) comprises a claw releasing connecting pipe (41) and a claw releasing handle (42), the claw releasing connecting pipe (41) has one end connected to the wire-locking claw assembly (3) and the other end connected to the claw releasing handle (42).

7. The valve delivery device according to claim 6, wherein the retraction assembly (2) further comprises a fixing base (22) and a retraction connecting pipe (23), an end of the fixing claw (21) not provided with the mounting groove (213) is connected to the fixing base (22), the claw releasing assembly (4) is sleeved on the retraction connecting pipe (23), and an end of the retraction connecting pipe (23) is connected to the fixing base (22).

8. The valve delivery device according to claim 7, wherein the retraction assembly (2) further comprises a mounting part (24), the mounting part (24) is located on a side of the fixing claw (21) not provided with the mounting groove (213), and the mounting part (24) is sleeved on the fixing base (22) and connected to the fixing claw (21).

9. The valve delivery device according to claim 7, wherein the valve delivery system comprises a receiving pipe (6) and a rotating wheel assembly (5), the receiving pipe (6) is sleeved on the retraction assembly (2) and configured to accommodate the valve, and the rotating wheel assembly (5) is configured to control movement of the receiving pipe (6) along an axial direction of the valve delivery system.

10. The valve delivery device according to claim 7, wherein the valve delivery system comprises a limit pin (7), the limit pin (7) is located at an end of the claw releasing connecting pipe (41) away from the wire-locking claw assembly (3), and is configured to limit movement of the claw releasing connecting pipe (41) along an axial direction of the valve delivery system.
